(19) Europäisches Patentamt
European Patent Office
Office européen des brevets

(11) Veröffentlichungsnummer: **0 213 071**
**B1**

(12) # EUROPÄISCHE PATENTSCHRIFT

(45) Veröffentlichungstag der Patentschrift: **08.03.89**

(21) Anmeldenummer: **86810335.9**

(22) Anmeldetag: **24.07.86**

(51) Int. Cl.⁴: **C 07 C 143/675**, C 07 C 147/12, C 09 B 62/006, C 07 D 251/50, C 07 D 251/44, C 07 D 239/42, C 07 D 251/42

(54) **Verfahren zur Herstellung faserreaktiver Azoverbindungen.**

(30) Priorität: **30.07.85 CH 3305/85**

(43) Veröffentlichungstag der Anmeldung: **04.03.87 Patentblatt 87/10**

(45) Bekanntmachung des Hinweises auf die Patenterteilung: **08.03.89 Patentblatt 89/10**

(84) Benannte Vertragsstaaten: **BE CH DE FR GB LI**

(56) Entgegenhaltungen:
**CH-A-536 353**
**DE-A-2 145 391**
**GB-A-1 166 913**

(73) Patentinhaber: **CIBA- GEIGY AG, Klybeckstrasse 141, CH- 4002 Basel (CH)**

(72) Erfinder: **Oxenius, Rüdiger, Dr., Brunnenweg 15, D-7888 Rheinfelden (DE)**
Erfinder: **Wilhelm, Charles, Annexe du Tannwald 14, F-68220 Leymen (FR)**

**EP 0 213 071 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein neues Verfahren zur Herstellung wasserlöslicher, faserreaktiver Azoverbindungen mit einer Phenylendiamin-Diazokomponente, deren eine freie Aminogruppe in Gegenwart von Salzen faserreaktiv acyliert wird.

Die bisher üblichen Herstellungsverfahren für Azoverbindungen mit einer faserreaktiven N-acylierten Phenylendiamin-Diazokomponente sind z. B.:

a) Kondensation eines Phenylendiamins mit einem den Reaktivrest einführenden Mittel, Reinigung dieses Zwischenproduktes und nachfolgende Diazotierung und Kupplung; oder

b) Kondensation eines Phenylendiamins mit einer Schutzgruppe wie z. B. Acetylchlorid, Reinigung dieses Zwischenproduktes, nachfolgende Diazotierung und Kupplung, anschliessende Abspaltung der Schutzgruppe und Kondensation der freien Aminogruppe mit einem den Reaktivrest einführenden Mittel.

Nachteil beider Verfahrensvarianten ist die notwendige Abtrennung des N-monoacylierten Phenylendiamins von den Ausgangsmaterialien und N,N-diacyliertem Phenylendiamin.

Nachteil der zweiten Verfahrensvariante ist ferner, dass Kupplungskomponenten, welche freie Aminogruppen enthalten, ebenfalls mit dem faserreaktiven Acylierungsmittel reagieren können.

Überraschenderweise wurde nun ein neues Verfahren gefunden, welches die genannten Nachteile nicht aufweist und welches erlaubt, auf einfache Art und Weise wasserlösliche Azoverbindungen mit einer faserreaktiven N-acylierten Phenylendiamin-Diazokomponente ohne Reinigung oder Isolierung der Zwischenprodukte herzustellen.

Gegenstand der vorliegenden Erfindung ist somit ein Verfahren zur Herstellung von Azoverbindungen der Formel

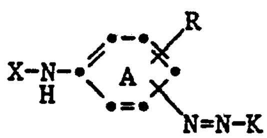

(1),

worin K der Rest einer Kupplungskomponente, R eine wasserlöslichmachende Gruppe und X ein faserreaktiver Rest ist und der Benzolring A gegebenenfalls weitersubstituiert ist, welches dadurch gekennzeichnet ist, dass man eine Verbindung der Formel

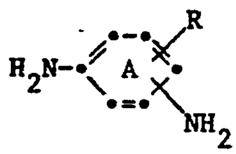

(2)

in wässrigem Medium, enthaltend 5 bis 25 Gewichtsprozent Alkalihalogenid oder Alkalisulfat, bezogen auf das Gewicht des Mediums, mit einer den Rest X einführenden Verbindung zu der Verbindung der Formel

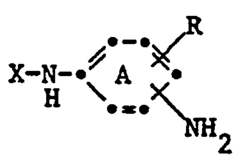

(3)

umsetzt, diese direkt diazotiert und auf eine Kupplungskomponente der Formel

H - K (4)

kuppelt, wobei A, R, X und K in den Formeln (2), (3) und (4) die unter Formel (1) angegebenen Bedeutungen haben.

Vorzugsweise ist der Benzolring A nicht weitersubstituiert.

Bei diesem Vorgehen kann man überraschenderweise auf die aufwendige Reinigung der Verbindung der Formel (3) verzichten, die Reaktionszeit über alle Verfahrensstufen deutlich verkürzen und die Azoverbindungen der Formel (1) in höherer Reinheit als nach den bisher üblichen Verfahren erhalten. Ferner wird durch die erfindungsgemässe Verfahrensweise eine deutliche Steigerung der Ausbeute erreicht.

Die in dem erfindungsgemässen Verfahren verwendbaren Phenylendiamine der Formel (2) sind vorzugsweise 1,3-Phenylendiamine und vor allem 1,4-Phenylendiamine, die eine wasserlöslichmachende Gruppe enthalten,

und die gegebenenfalls weitersubstituiert sind wie z. B. durch $C_{1-4}$-Alkyl, wie Methyl, $C_{1-4}$-Alkoxy, wie Methoxy, oder Halogen wie Fluor, Chlor oder Brom. Als Beispiele seien genannt: 1,3-Phenylendiamin-4-sulfonsäure, 6-Methoxy-1,3-phenylendiamin-4-sulfonsäure, 2-Methylphenylsulfonyl-1,4-phenylendiamin, 2-Ureido-1,4-phenylendiamin, 1,4-Phenylendiamin-2-sulfonsäure, 2,5-Diamino-4'-methyl-1,1-diphenylsulfon-3-sulfonsäure.

Als wasserlöslichmachende Gruppe R in Formel (2) kommt eine der folgenden Gruppen in Betracht: eine Sulfon-, Sulfonamid-, N-Mono- oder N,N-Dialkylsulfonamidgruppe oder eine Carboxy- oder Ureidogruppe sowie insbesondere die Sulfonsäuregruppe.

Als Sulfongruppe kommt die Alkylsulfon-, insbesondere $C_{1-4}$-Alkylsul-fongruppe oder die Arylsulfongruppe, insbesondere die Phenylsulfongruppe, in Betracht, wobei der Phenylring gegebenenfalls weiter substituiert sein kann, z. B. durch $C_{1-4}$-Alkyl, wie Methyl, $C_{1-4}$-Alkoxy, wie Methoxy, Halogen wie Fluor, Chlor oder Brom oder Sulfo.

Als N-Mono- oder N,N-Dialkylsulfonamidgruppe kommt insbesondere eine solche mit einem oder zwei $C_{1-4}$-Alkylresten in Betracht.

Gemäss dem erfindungsgemässen Verfahren wird die Acylierung des Phenylendiamins der Formel (2) mit einer den Rest X einführenden Verbindung in Gegenwart eines der genannten Salze durchgeführt. Vorzugsweise wird das erfindungsgemässe Verfahren in Gegenwart weiterer zusätzlicher Salze durchgeführt. Die verwendbaren Salze sind insbesondere Mischungen aus Alkalihalogenid oder Alkalisulfat und einem vorzugsweise säurebindenden Salz, wie Alkaliacetat, Alkalihydroxid, Alkalicarbonat oder Alkalihydrogencarbonat oder deren Mischungen.

Als Alkalihalogenide kommen z. B. in Betracht: Lithium-, Natrium- oder Kaliumfluorid, Lithium-, Natrium- oder Kaliumchlorid, Lithium-, Natrium- oder Kaliumbromid, Lithium-, Natrium- oder Kaliumjodid.

Als Alkalisulfate kommen z. B. in Betracht: Natrium- oder Kaliumsulfat.

Als säurebindende Salze kommem z. B. in Betracht: Natriumacetat, Natrium- oder Kaliumhydroxid, Natrium- oder Kaliumcarbonat, Natrium- oder Kaliumhydrogencarbonat. Vorzugsweise wird in dem erfindungsgemässen Verfahren eine Mischung aus Natriumacetat und Natriumhydrogencarbonat verwendet.

Die Mengen, in denen die saurebindenden Salze in dem erfindungsgemässem Verfahren verwendet werden, können in weiten Grenzen schwanken, im allgemeinen richten sich die Mengen nach der eingesetzten Menge des Acylierungsmittels bzw. nach der während der Acylierung freiwerdenden Säuremenge. Ein Überschuss an säurebindenden Salzen hat sich im allgemeinen als vorteilhaft erwiesen.

Die Menge an Alkalihalogenid oder Alkalisulfat, die in dem erfindungsgemässen Verfahren verwendet wird, richtet sich nach Volumen bzw. dem Gewicht des wässrigen Mediums des Phenylendiamins der Formel (2), d.h. die Menge an Alkalihalogenid oder Alkalisulfat ist unabhängig von der zuvor gelösten Menge an Phenylendiamin. Im allgemeinen haben sich 5 bis 25 Gewichtsprozent Alkalihalogenid oder Alkalisulfat, bezogen auf das Gewicht des wässrigen Mediums, als vorteilhaft erwiesen. Vorzugsweise werden in dem erfindungsgemässen Verfahren 8 bis 13 Gewichtsprozent Alkalihalogenid oder Alkalisulfat verwendet.

Als Acylierungsmittel, die ausser der acylierenden Stelle noch einen vorzugsweise aliphatischen, aromatischen oder heterocyclischen Reaktivrest X enthalten, kommen insbesondere die Halogenide oder Anhydride organischer Säuren sowie Heterocyclen in Betracht, die leicht austauschbare Atome oder Atomgruppen enthalten.

Im Anschluss an die Acylierung der Verbindung der Formel (2) ist keine weitere Aufarbeitung der Reaktionsmasse oder -suspension, welche das Produkt der Formel (3) enthält, erforderlich. Das Produkt der Formel (3) kann sofort diazotiert und auf eine Kupplungskomponente der Formel (4) gekuppelt werden. Die Diazotierung der Verbindung der Formel (3) erfolgt nach an sich bekannten Methoden, wie z. B. durch Einwirkung salpetriger Säure in wässrig-mineralsaurer Lösung bei tiefer Temperatur; die Kupplung auf die Kupplungskomponente der Formel (4) erfolgt bei sauren, neutralen bis alkalischen pH-Werten.

Als Kupplungskomponenten der Formel (4) kommen insbesondere solche der Benzol-, Naphthalin- oder der heterocyclischen Reihe in Betracht.

Im Anschluss an die Kupplungsreaktion wird die Azoverbindung der Formel (1) in an sich bekannter Weise isoliert, wie z. B. durch Aussalzen, Abfiltrieren und anschliessender Trocknung.

Eine Umwandlungsreaktion, die in manchen Fällen an die Synthese angeschlossen werden kann, ist beispielsweise, dass man einen Reaktivfarbstoff der Formel (1), welcher z. B. einen α,β-Dibrompropionylamino-Rest enthält, mit halogenwasserstoffabspaltenden Mitteln, wie z. B. Natriumhydroxid, behandelt, so dass die α,β-Dibrompropionylgruppe in die α-Bromacryloylgruppe umgewandelt wird.

Bevorzugte Ausführungsformen des erfindungsgemässen Verfahrens sind dadurch gekennzeichnet, dass

a) das Gewichtsverhältnis von Wasser zu der Verbindung der Formel (2) 3 : 1 bis 20 : 1, insbesondere 4 : 1 bis 8 : 1 ist;

b) man 8 bis 13 Gewichtsprozent Alkalihalogenid oder Alkalisulfat, insbesondere Natriumchlorid verwendet;

c) man ein gepuffertes Gemisch von Salzen verwendet, insbesondere eine Mischung von Alkalicarbonat, wie z. B. $Na_2CO_3$ oder $K_2CO_3$, oder Alkalihydrogencarbonat, wie z. B. $NaHCO_3$ oder $KHCO_3$, und Alkaliacetat, wie z. B. Natriumacetat oder Kaliumacetat, und Alkalihalogenid oder Alkalisulfat verwendet;

d) man eine Verbindung der Formel (2) mit einer den Rest X einführenden Verbindung bei 0° bis 20°C und einem pH-Wert von 3 bis 9 umsetzt;

e) man adiabatisch diazotiert bei einer Temperatur von 0° bis 70° C, insbesondere 0° bis 50°, vorzugsweise 20° bis 50° C, ganz besonders bevorzugt 20° bis 40° C;
f) man adiabatisch kuppelt.

Weitere bevorzugte Ausführungsformen des erfindungsgemässen Verfahrens sind dadurch gekennzeichnet,

g) dass man eine Verbindung der Formel (2) verwendet, worin R eine Sulfophenylsulfon- oder insbesondere Sulfonsäuregruppe ist; ganz besonders bevorzugt verwendet man als Verbindung der Formel (2) eine 1,3-Phenylendiaminsulfonsäure, insbesondere 1,3-Phenylendiamin-4-sulfonsäure und vor allem 1,4-Phenylendiamin-2-sulfonsäure; eine weitere interessante Verbindung der Formel (2) ist ferner 2,5-Diamino-4'-methyl-1,1'-diphenylsulfon-3'-sulfonsäure;
h) dass man als den Rest X einführende Verbindung α,β-Dibrompropionsäurechlorid, α,β-Dichlorpropionsäurechlorid oder α-Chloracrylsäurechlorid verwendet; weitere interessante Verbindungen sind Bromessigsäureanhydrid insbesondere Chloressigsäureanhydrid oder Chloracetylchlorid.
i) dass man als Kupplungskomponénte der Formel (4) eine Kupplungskomponente der Benzol-, Naphthalin- oder der heterocyclischen Reihe verwendet.

Insbesondere ist K der Rest eines Amino- oder Alkoxybenzols, Amino- oder Alkoxynaphthalins, Naphthols, Aminonaphthols, Pyrazolons, Aminopyrazols, Pyridons, Pyrimidins, Indols, Naphthylimidazols, Diphenylamins, Pyrazolo[2,3-a]pyrimidins, Tetrahydrochinolins oder Acetessigsäureamids, wobei die oben genannten Reste weitersubstituiert sein können, durch die bei Azofarbstoffen üblichen Substituenten: z. B. Alkylgruppen mit 1 bis 4 Kohlenstoffatomen, wie Methyl, Äthyl, Propyl, Isopropyl, Butyl, Isobutyl, sek.-Butyl und tert.-Butyl, Alkoxygruppen mit 1 bis 4 Kohlenstoffatomen, wie Methoxy, Äthoxy, Propyloxy, Isopropyloxy, Butyloxy, Isobutyloxy, sek.-Butyloxy und tert.-Butyloxy, Phenoxy, Alkanoylaminogruppen mit 1 bis 6 Kohlenstoffatomen, wie Acetylamino oder Propionylamino, Benzoylamino, Aminogruppen, wie $-NH_2$, Methylamino, Äthylamino, Dimethylamino, Diäthylamino, Cyanäthylamino, Hydroxyäthylamino, Dihydroxyäthylamino, Cyclohexylamino, Benzylamino und Phenylamino, Carbonsäureestergruppen, wie Methoxycarbonyl und Äthoxycarbonyl, Trifluormethyl, Nitro, Cyan, Acetyl, Methylsulfonyl, Carbamoyl, Sulfamoyl, Ureido, Hydroxy, Carboxy, Sulfo, Sulfomethyl und Halogen, wie Fluor, Chlor und Brom, sowie faserreaktive Reste.

Bevorzugte Beispiele für Kupplungskomponenten der Formel (4) sind: 2-Amino-8-naphthol-6-sulfonsäure, 2-Methylamino-8-naphthol-6-sulfonsäure, 1-(2'-5'-Dichlor-4-sulfophenyl)-3-methyl-5-pyrazolon, 1-(2'-Chlor-6'-methylphenyl)-3-methyl-5-pyrazolon, 1-Hydroxy-3-sulfo-6-N-methyl-N-dibrompropionylaminonaphthalin, 2-Aminonaphthalin-6-sulfonsäure, 1-(3'- oder 4'-Dibrompropionylaminobenzoylamino)-8-hydroxynaphthalin-4,6- oder -3,6-disulfonsäure, 2-α-Brom-acryloyl-amino-8-hydroxynaphthalin-6-sulfonsäure, 1-Acetylamino-8-hydroxynaphthalin-4,6-disulfonsäure, 2-Amino-8-hydroxynaphthalin-6-N-methyl-N-phenylsulfonsäureamid.

Ganz besonders bevorzugt verwendet man eine Aminonaphtholsulfonsäure.

Eine ganz besonders bevorzugte Ausführungsform des erfindungsgemässen Verfahrens zur Herstellung der Azoverbindung der Formel

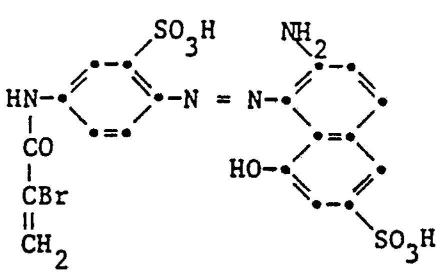

(5)

ist dadurch gekennzeichnet, dass nan 1,4-Phenylendiamin-2-sulfonsäure mit α,β-Dibrompropionsäurechlorid umsetzt und die erhaltene Verbindung ohne Isolierung direkt diazotiert und auf 2-Amino-8-hydroxynaphthalin-6-sulfonsäure kuppelt und anschliessend mit einem bromwasserstoffabspaltenden Mittel umsetzt.

Eine ebenfalls ganz besonders bevorzugte Ausführungsform des erfindungsgemässen Verfahrens zur Herstellung der Azoverbindung der Formel

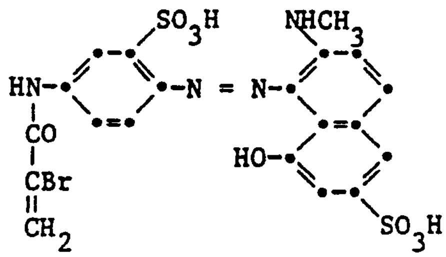

(6)

ist dadurch gekennzeichnet, dass man 1,4-Phenylendiamin-2-sulfonsäure mit α,β-Dibrompropionsäurechlorid umsetzt und die erhaltene Verbindung ohne Isolierung direkt diazotiert und auf 2-N-Methylamino-8-hydroxynaphthalin-6-sulfonsäure kuppelt und anschliessend mit einem bromwasserstoffabspaltenden Mittel umsetzt.

Interessant ist ferner die analoge Umsetzung von 1,3-Phenylendiamin-4-sulfonsäure mit Chloressigsäureanhydrid oder -chlorid, Diazotierung und Kupplung in 1-Stellung auf 2-Amino-8-hydroxynaphthalin-6-sulfonsäure-N-methyl-N-phenylamid.

Eine ebenfalls ganz besonders bevorzugte Ausführungform des erfindungsgemässen Verfahrens zur Herstellung des Azofarbstoffes der Formel

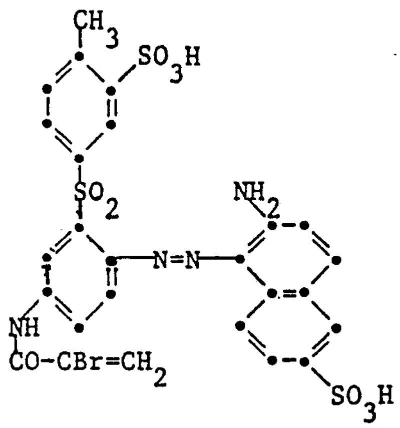

ist dadurch gekennzeichnet, dass man 2,5-Diamino-4'-methyl-1,1'-diphenylsulfon-3'-sulfonsäure mit α,β-Dibrompropionsäurechlorid umsetzt und die erhaltene Verbindung ohne Isolierung oder Reinigung direkt diazotiert und auf 2-Aminonapthalin-6-sulfonsäure kuppelt und anschliessend mit einem bromwasserstoffabspaltenden Mittel umsetzt.

Die in dem erfindungsgemässen Verfahren verwendeten Ausgangsverbindungen der Formeln (2) und (4) und die faserreaktiven Acylierungsmittel sind an sich bekannt und werden nach bekannten Methoden hergestellt.

Die gemäss dem erfindungsgemässen Verfahren herstellbaren sulfogruppenhaltigen Azoverbindungen liegen entweder in Form ihrer freien Sulfonsäure oder vorzugsweise als deren Salze vor.

Als Salze kommen beispielsweise die Alkali-, Erdalkali- oder Ammoniumsalze oder die Salze eines organischen Amins in Betracht. Als Beispiele seien die Natrium-, Lithium-, Kalium- oder Ammoniumsalze oder das Salz des Triäthanolamins genannt.

Als Beispiele für Acylierungsmittel, die erfindungsgemäss verwendet werden können, seien die folgenden genannt:

Brom- oder Chloressigsäureanhydrid,
Chlor- oder Bromacetylchlorid,
β-Chlor- oder β-Brompropionylchlorid,
α,β-Dichlor- oder α,β-Dibrompropionylchlorid,
Chlormaleinsäureanhydrid,
Carbylsulfat,
Acryloylchlorid,
β-Chlor- oder β-Bromacryloylchlorid,
α-Chloracryloylchlorid,
α,β-Dichlor- oder α,β-Dibromacryloylchlorid,
Trichloracryloylchlorid,
Chlorcrotonylchlorid,
Propiolsäurechlorid,

3,5-Dinitro-4-chlorbenzol-sulfonsäure- oder -carbonsäurechlorid,
3-Nitro-4-chlorbenzol-sulfonsäure- oder -carbonsäurechlorid,
2,2,3,3-Tetrafluorcyclobutan-1-carbonsäurechlorid,
2,2,3,3-Tetrafluorcyclobutyl-acrylsäurechlorid,
β-Chloräthylsulfonyl-endomethylen-cyclohexancarbonsäurechlorid,
Acrylsulfonyl-endomethylen-cyclohexancarbonsäurechlorid,
2-Chlorbenzoxazolcarbonsäurechloride,
2-Chlorbenzthiazolcarbon- oder -sulfonsäurechloride,
4,5-Dichlor-1-phenylpyridazoncarbon- oder -sulfonsäurechlorid,
4,5-Dichlorpyridazolpropionsäurechlorid,
1,4-Dichlorphthalazincarbon- oder -sulfonsäurechlorid,
2,3-Dichlorchinoxalincarbon- oder -sulfonsäurechlorid,
2,4-Dichlorchinazolincarbon- oder -sulfonsäurechlorid,
2-Methansulfonyl-4-chlor-6-methylpyrimidin,
2,4-Bis-methansulfonyl-6-methylpyrimidin,
2,4,6-Tri- oder 2,4,5,6-Tetrachlorpyrimidin,
2,4,6-Tri- oder 2,4,5,6-Tetrabrompyrimidin,
2-Methansulfonyl-4,5-dichlor-6-methylpyrimidin,
2,4-Dichlorpyrimidin-5-sulfonsäure,
5-Nitro- oder 5-Cyan-2,4,6-trichlorpyrimidin,
2,6-Bis-methansulfonylpyridin-4-carbonsäurechlorid,
2,4-Dichlor-5-chlormethyl-6-methyl-pyrimidin,
2,4-Dibrom-5-brommethyl-6-methyl-pyrimidin,
2,4-Dichlor-5-chlormethylpyrimidin,
2,4-Dibrom-5-brommethylpyrimidin,
2,5,6-Trichlor-4-methylpyrimidin,
2,6-Dichlor-4-trichlormethylpyrimidin,
2,4-Bismethylsulfonyl-5-chlor-6-methylpyrimidin,
2,4,6-Trimethylsulfonyl-1,3,5-triazin,
2,4-Dichlorpyrimidin,
3,6-Dichlorpyridazin,
3,6-Dichlorpyridazin-5-carbonsäurechlorid,
2,6-Dichlor- oder 2,6-Dibrom-4-carboäthoxypyrimidin,
2,4,5-Trichlorpyrimidin,
2,4-Dichlorpyrimidin-6-carbonsäurechlorid,
2,4-Dichlorpyrimidin-5-carbonsäurechlorid,
2,6-Dichlor- oder 2,6-Dibrompyrimidin-4- oder -5-carbonsäure- oder -sulfonsäureamide bzw. -4- oder -5-sulfonsäurechlorid,
2,4,5,6-Tetrachlorpyridazin,
5-Brom-2,4,6-trichlorpyrimidin,
5-Acetyl-2,4,6-trichlorpyrimidin,
5-Nitro-6-methyl-2,4-dichlorpyrimidin,
2-Chlorbenzthiazol-6-carbonsäurechlorid,
2-Chlorbenzthiazol-6-sulfonsäurechlorid,
5-Nitro-6-methyl-2,4-dichlorpyrimidin,
2,4,6-Trichlor-5-brompyrimidin,
2,4,5,6-Tetrafluorpyrimidin,
4,6-Difluor-5-chlorpyrimidin,
2,4,6-Trifluor-5-chlorpyrimidin,
2,4,5-Trifluorpyrimidin,
2,4,6-Trichlor- (-Tribrom- oder -Trifluor)-s-triazin, sowie 4,6-Dichlor- (-Dibrom- oder -Difluor)-s-triazine, die in 2-Stellung substituiert sind durch einen Aryl- oder Alkylrest, z. B. einen Phenyl-, Methyl- oder Äthylrest, oder durch den Rest einer aliphatischen oder aromatischen, über das Schwefelatom gebundenen Mercapto- bzw. über das Sauerstoffatom gebundenen Hydroxylverbindung oder insbesondere durch eine -$NH_2$ Gruppe oder durch den Rest einer über das Stickstoffatom gebundenen aliphatischen, heterocyclischen oder aromatischen Aminoverbindung.

Die in 2-Stellung substituierten 4,6-Dihalogen-s-triazine werden z. B. durch Umsetzung von Trihalogen-s-triazinen mit den erwähnten Amino-, Hydroxy- oder Mercaptoverbindungen erhalten. Der Substituent in 2-Stellung eines 4,6-Dihalogen-s-triazins kann z. B. der Rest einer der im folgenden genannten Amino-, Hydroxy- und Mercaptoverbindungen sein;

Ammoniak, Methylamin, Dimethylamin, Äthylamin, Diäthylamin, Propylamin, Isopropylamin, Butylamin, Dibutylamin, Isobutylamin, sek.-Butylamin, tert.-Butylamin, Hexylamin, Methoxyäthylamin, Äthoxyäthylamin, Methoxypropylamin, Chloräthylamin, Hydroxyäthylamin, Dihydroxyäthylamin, Hydroxypropylamin, Aminoäthansulfonsäure, β-Sulfatoäthylamin, Benzylamin, Cyclohexylamin, Anilin, o-, m- und p-Toluidin, 2,3-, 2,4-, 2,5-, 2,6-, 3,4- und 3,5-Dimethylanilin, o-, m- und p-Chloranilin, N-Methylanilin, N-Äthylanilin, 3- oder 4-Acetylaminoanilin, 2,5-Dimethoxyanilin, o-, m- und p-Anisidin, o-, m- und p-Phenetidin, Naphthylamin-(1) Naphthylamin-(2), 2-amino-1-hydroxy-naphthalin, 1-Amino-4-hydroxynaphthalin, 1-Amino-8-hydroxy-naphthalin, 1-Amino-2-hydroxynaphthalin, 1-Amino-7-hydroxy-naphthalin, Orthanilsäure, Metanilsäure, Sulfanilsäure, Anilin-2,4-disulfonsäure, Anilin-2,5-disulfonsäure, Anthranilsäure, m- und p-Aminobenzoesäure, 2-Aminotoluol-4-sulfonsäure, 2-Aminotoluol-5-sulfonsäure, p-Aminosalicylsäure, 1-Amino-4-carboxybenzol-3-sulfonsäure, 1-Amino-2-carboxybenzol-5-sulfonsäre, 1-Amino-5-carboxybenzol-2-sulfonsäure, 1-Naphthylamin-2-, -3-, -4-, -5-, -6-, -7- und 8-sulfonsäure, 2-Naphthylamin-1-, -3-, -4-, -5-, -6-, -7- und -8-sulfonsäure, 1-Naphthylamin-2,4-, -2,5-, -2,7-, -2,8-, -3,5-, -3,6-, -3,7-, -3,8-, -4,6-, -4,7-, -4,8- und -5,5-disulfonsäure, 2-Naphthylamin-1,5-, -1,6-, -1,7-, -3,6-, -3,7-, -4,7-, -4,8-, -5,7- und -6,8-disulfonsäure, 1-Naphthylamin-2,4,6-,-2,4,7-, -2,5,7-, -3,5,7-, -3,6,8- und -4,6,8-trisulfonsäure, 2-Naphthylamin-1,3,7-, -1,5,7-, -3,5,7-, -3,6,7-, -3,6,8- und -4,6,8-trisulfonsäure, 2-, 3- und 4-Aminopyridin, 2-Aminobenzthiazol, 5-, 6- und 8-Aminochinolin, 2-Aminopyrimidin, Morpholin, Piperidin, Piperazin, Wasser, Methanol, Äthanol, Propanol, Isopropanol, n-Butanol, Isobutanol, sek.-Butanol, tert.-Butanol, Hexanol, Cyclohexanol, β-Methoxyäthanol, β-Äthoxyäthanol, γ-Methoxypropanol, γ-Äthoxypropanol, β-Äthoxy-β-äthoxy-äthanol, Glycolsäure, Phenol, o-, m- und p-Chlorphenol, o-, m- und p-Nitrophenol, o-, m- und p-Hydroxybenzoesäure, o-, m- und p-Phenolsulfonsäure, Phenol-2,4-disulfonsäure, α-Naphthol, β-Naphthol, 1-Hydroxynaphthalin-8-sulfonsäure, 2-Hydroxynaphthalin-1-sulfonsäure, 1-Hydroxynaphthalin-5-sulfonsäure, 1-Hydroxynaphthalin-4-sulfonsäure, 1-Hydroxynaphthalin-6- oder -7-sulfonsäure, 2-Hydroxynaphthalin-6-, -7- oder -8-sulfonsäure, 2-Hydroxynaphthalin-4-sulfonsäure, 2-Hydroxynaphthalin-4,8- oder -6,8-disulfonsäure, 1-Hydroxynaphthalin-4,8-disulfonsäure, 2-Hydroxynaphthalin-3,6-disulfonsäure, Methanthiol, Äthanthiol, Propanthiol, Isopropanthiol, n-Butanthiol, Thioglykolsäure, Thioharnstoff, Thiophenol, α-Thionaphthol, β-Thionaphthol.

Die Einführung des in 2-Stellung des Triazinrestes stehenden Substituenten kann auch nach der Diazotierung und Kupplung geschehen. Man kann somit z. B. eine der oben genannten Amino-, Hydroxy- oder Mercaptoverbindungen nachträglich mit einem bereits an den Azofarbstoff gebundenen Dihalogen-s-triazinrest kondensieren.

Wertvolle Amino-fluortriazine sind z. B.:

2-Amino-4,6-difluor-s-triazin, 2-Phenylamino-4,6-difluor-s-triazin, 2-N-Methyl-N-phenylamino-4,6-difluor-s-triazin, 2-(Methylphenylamino)-4,6-difluor-s-triazin, 2-(Chlorphenylamino)-4,6-difluor-s-triazin, 2-(Sulfophenylamino)-4,6-difluor-s-triazin, 2-N-Methyl-N-(methylphenylamino)-4,6-difluor-s-triazin, 2-N-Methyl-N-(chlorphenylamino)-4,6-difluor-s-triazin, 2-N-Methyl-N-(sulfophenylamino)-4,6-difluor-s-triazin und 2-(Chloräthylsulfonyläthylamino)-4,6-difluor-s-triazin.

Beispiele für Kupplungskomponenten der Formel (4), die in dem erfindungsgemässen Verfahren ausser den bereits genannten verwendet werden können, sind in grosser Zahl aus der Literatur bekannt, wie z. B. Dimethylanilin, Diäthylanilin, 3-Methyl-dimethylanilin, 3-Methyl-diäthylanilin, 3-Acetylamino- oder 3-Methoxycarbonylamino- oder 3-Ureido-dimethylanilin, 3-Methyl-6-methoxy-diäthylanilin, 2,5-Dimethoxydiäthylanilin, N-Äthyl-N-benzylanilin, N-Äthyl-N-(β-cyanäthyl)anilin, N-Äthyl-N-(β-hydroxyäthyl)anilin, N-Äthyl-N-(β-acetoxyäthyl)anilin, N,N-Dibutylanilin, 3-(α,β-Dibrompropionylamino)-N,N-dimethyl- oder -diäthylanilin, 3-(α-Chloracetylamino)- oder 3-(α-Bromacryloylamino)-N,N-dimethylanilin, 1-Hydroxy-7-amino-3-sulfonaphthalin, 1-Hydroxy-7-methylamino- oder -7-phenylamino-3-sulfonaphthalin, 1-Hydroxy-7-(α,β-dibrompropionylamino)- oder 7-(α-Chloracetylamino)-3-sulfonaphthalin, 1-Hydroxy-8-amino-3,6- oder -3,5-disulfonaphthalin, 1-Hydroxy-8-benzoylamino-3,6- oder -3,5-disulfonaphthalin, 1-Hydroxy-8-ureido-3,6- oder -3,5-disulfonaphthalin, 1-Hydroxy-8-acetylamino-3,6- oder -3,5-disulfonaphthalin, 1-Hydroxy-8-(3'-α,β-dibrompropionylaminobenzoylamino)-3,6- oder -3,5-disulfonaphthalin, 1-(2',3'- oder 4'-Sulfophenyl)-3-methylpyrazolon-5, 1-(2'-Chlor-4'- oder 5'-sulfophenyl)-3-methylpyrazolon-5, 1-(2'-Methyl-4'-sulfophenyl)-3-methylpyrazolon-5, 1-[4',8'-Disulfonaphthyl-(2)]-3-methylpyrazolon-5, 1-[5',7'-Disulfonaphthyl-(2)-3-methylpyrazolon-5, 1-(2'-Chlor-5'-sulfophenyl)-3-methyl-5-aminopyrazol, 1-(2'-Chlor-4'-sulfophenyl)-3-methyl-5-aminopyrazol, 1-(3'- oder 4'-sulfophenyl)-3-methyl-5-aminopyrazol, 1-Äthyl-3-cyano-4-methyl-6-hydroxy-pyridon-2, 1-Äthyl-4-methyl-6-hydroxy-pyridon-2, 2-Methylindol, 2-Phenylindol.

Gegenstand der Erfindung ist ferner ein Verfahren zur Herstellung von Verbindungen der Formel (3), welches dadurch gekennzeichnet ist, dass man eine Verbindung der Formel (2) in wässrigem Medium, enthaltend 5 bis 25 Gewichtsprozent Alkalihalogenid oder Alkalisulfat, bezogen auf das Gewicht des Mediums, mit einer den Rest x einführenden Verbindung zu dem Produkt der Formel (3) umsetzt.

Ausgangsprodukte, Verfahrensbedingungen und bevorzugte Verfahrensweisen entsprechen dem vorstehend beschriebenen Reaktionsschritt zur Acylierung eines Phenylendiamins. Die erfindungsgemäss erhaltenen Verbindungen der Formel (3) eignen sich z. B. als Diazokomponenten zur Herstellung von Azoverbindungen.

Die nach dem erfindungsgemässen Verfahren erhaltenen Azoverbindungen der Formel (1) eignen sich als Farbstoffe zum Färben oder Bedrucken textiler Fasermaterialien, insbesondere polyhydroxylierten Materialien faseriger Struktur wie cellulosehaltiger Stoffe, z. B. regenerierte Cellulose, Leinen und vor allem Baumwolle. Sie eignen sich besonders zum Färben oder Bedrucken stickstoffhaltiger Textilmaterialien wie Seide oder vor allem Wolle sowie Superpolyamid- oder Superpolyurethanfasern aus schwach alkalischem, neutralem oder saurem Bade, z. B. aus essigsäurem Bad.

Das erfindungsgemässe Verfahren weist gegenüber den bekannten Verfahren zur Herstellung der Azoverbindungen der Formel (1) neben den genannten noch folgende Vorteile auf. Durch die selektive Kondensation der Phenylendiamin-Verbindungen mit dem Acylierungsmittel auch in heterogener Phase kann

die Umsetzung konzentrierter erfolgen als es bisher möglich war, was eine Einsparung an Lösungsmittel und Energie und eine geringere Menge Abwasser zur Folge hat. Ferner wird mit dem erfindungsgemässen Verfahren eine geringere Abwasserbelastung erreicht. Durch die höhere Reinheit der gemäss dem erfindungsgemässen Verfahren erhaltenen Azoverbindungen verglichen mit den auf übliche Weise hergestellten gleichen Azoverbindungen kann in einigen Fällen eine Verbesserung der Echtheiten der erhaltenen Färbungen erzielt werden.

Die nachfolgenden Beispiele dienen der Veranschaulichung der Erfindung. Darin sind die Teile Gewichtsteile und die Prozente Gewichtsprozente. Die Temperaturen sind in Celsiusgraden angegeben. Die Beziehung zwischen Gewichtsteilen und Volumenteilen ist dieselbe die diejenige zwischen Gramm und Kubikzentimeter.

**Beispiel 1:**

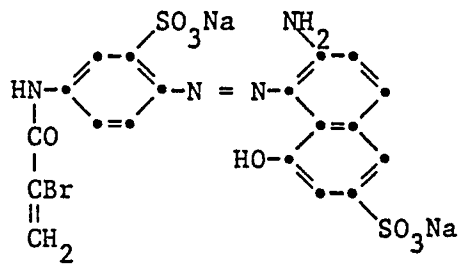

53 Teile 1,4-Phenylendiamin-2-sulfonsäure werden in 80 Teilen Wasser verrührt, auf 50° erwärmt und durch Zugabe von wässriger Natriumhydroxid-Lösung bei pH 8 gelöst. Das Volumen wird mit Wasser auf 250 Teile gestellt. Nach Zugabe von ca. 150 Teilen Eiswerden der Lösung bei einer Temperatur von 0 bis 3° 45 Teile Natriumchlorid und anschliessend 15 Teile Natriumacetat sowie 20 Teile Natriumhydrogencarbonat unter Rühren zugesetzt. Danach werden 67 Teile 2,3-Dibrom-propionsäurechlorid portionenweise zugegeben. Durch Zugabe von Eis wird eine Temperatur von 5 bis 10° eingehalten. Anschliessend wird mit Wasser auf ein Volumen von 800 Teilen eingestellt und bei ca. 30° unter starkem Rühren 60 Teile 32-%-ige Salzsäure zugesetzt. Bei adiabatischer Temperaturführung wird mit 62,5 Teilen 4N Natriumnitrit-Lösung diazotiert. Ein eventuell vorhandener Nitritüberschuss kann mit Sulfaminsäure zerstört werden.

66 Teile 2-Amino-8-hydroxynaphthalin-6-sulfonsäure werden in 100 Teilen Wasser unter Zugabe von 27,5 Teilen 30-%-iger Natriumhydroxid-Lösung gelöst und durch Zugabe von Wasser auf ein Volumen von 350 Teilen gestellt. Diese Lösung fügt man der oben hergestellten Diazosuspension hinzu und stellt mit 30-%-iger Natriumhydroxid-Lösung auf pH 3,8. Zur Vervollständigung der Kupplung werden dann 15 Teile Natriumacetat zugegeben. Nach beendeter Kupplung wird die Farbstofflösung durch Zugabe von 50 Teilen 30-%-iger Natriumhydroxid-Lösung auf pH 11,5 bis 12 gestellt, wobei die Temperatur zwischen 25 und 30° gehalten wird. Man lässt 15 Minuten bei pH 11,5 bis 12 rühren und neutralisiert dann auf pH 6 durch Zugabe von 32-%-iger Salzsäure. Anschliessend wird die Suspension filtriert und im Vakuum bei 70 bis 80° getrocknet. Man erhält ein blaurotes Pulver, welches sich in Wasser löst und Wolle aus essigsaurem Bad in sehr nassechten, blaustichig roten Tönen färbt. Die Ausbeute über alle Stufen beträgt 90 %.

**Beispiel 2:**

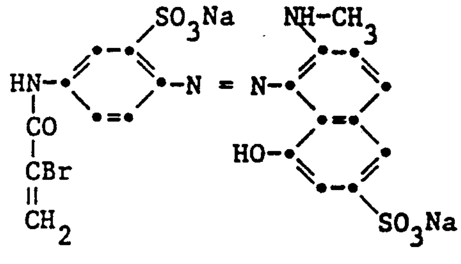

53 Teile 1,4-Phenylendiamin-2-sulfonsäure werden in 80 Teilen Wasser verrührt, auf 50° erwärmt und durch Zugabe von wässriger Natriumhydroxid-Lösung bei pH 8 gelöst. Das Volumen wird mit Wasser auf 250 Teile gestellt. Nach Zugabe vom Eis (ca. 150 Teile) werden der Lösung bei einer Temperatur von 0 bis 3° 45 Teile

Natriumchlorid und anschliessend 15 Teile Natriumacetat sowie 20 Teile Natriumhydrogencarbonat unter Rühren zugesetzt. Danach werden 67 Teile 2,3-Dibrompropionsäurechlorid portionenweise zugegeben. Durch Zugabe von Eis wird eine Temperatur von 5 bis 10° eingehalten. Anschliessend wird mit Wasser auf ein Volumen vom 800 Teilen eingestellt und bei ca. 30° unter starkem Rühren 60 Teile 32-%-ige Salzsäure zugesetzt. Bei adiabatischer Temperaturführung wird mit 62,5 Teilen 4N Natriumnitrit-Lösung diazotiert. Ein eventuell vorhandener Nitritüberschuss kann mit Sulfaminsäure zerstört werden.

70 Teile 2-N-Methylamino-8-hydroxynaphthalin-6-sulfonsäure werden in 100 Teilen Wasser unter Zugabe von 27,5 Teilen 30-%-iger Natriumhydroxid-Lösung gelöst und durch Zugabe von Wasser auf ein Volumen von 350 Teilen gestellt. Diese Lösung fügt man der oben hergestellten Diazosuspension hinzu und stellt mit 30-%-iger Natriumhydroxid-Lösung auf pH 3,8. Zur Vervollständigung der Kupplung werden dann 15 Teile Natriumacetat zugegeben. Nach beendeter Kupplung wird die Farbstofflösung durch Zugabe von 50 Teilen 30-%-iger Natriumhydroxid-Lösung auf pH 11,5 bis 12 gestellt, wobei die Temperatur zwischen 25 und 30° gehalten wird. Man lässt 15 Minuten bei pH 11,5 bis 12 rühren und neutralisiert dann auf pH 6 durch Zugabe von 32-%-iger Salzsäure. Anschliessend wird die Suspension filtriert und im Vakuum bei 70 bis 80° getrocknet. Man erhält ein blaurotes Pulver, welches sich in Wasser löst und Wolle aus essigsaurem Bad in sehr nassechten, blaustichig roten Tönen färbt. Die Ausbeute über alle Stufen beträgt 90 %.

**Beispiel 3:**

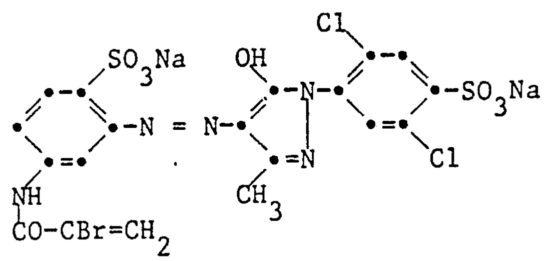

53 Teile 1,3-Phenylendiamin-4-sulfonsäure werden in 80 Teilen Wasser verrührt, auf 60° erwärmt und durch Zugabe von wässriger Natriumhydroxid-Lösung bei pH 7,5 gelöst. Anschliessend stellt man mit Wasser auf ein Volumen von 230 Teilen. Nach Zugabe von Eis werden der Lösung bei einer Temperatur von 0 bis 3° 25 Teile Natriumchlorid unter Rühren zugesetzt. Danach werden 70 Teile 2,3-Dibrompropionsäurechlorid portionenweise zugegeben. Durch Zugabe von Eis wird eine Temperatur von 0 bis 10° eingehalten und der pH wird durch Zulauf von Natriumhydroxid-Lösung bei pH 5,0 konstant gehalten. Anschliessend wird mit Wasser auf ein Volumen von 700 Teilen eingestellt und bei ca. 15° unter starkem Rühren 60 Teile 32-%-ige Salzsäure zugesetzt. Bei adiabatischer Temperaturführung wird mit 64 Teilen 4N Natriumnitrit-Lösung diazotiert. Ein eventuell vorhandener Nitritüberschuss kann mit Sulfaminsäure zerstört werden.

85,6 Teile (2,5-Dichlor-4-sulfo)-phenyl-3-methylpyrazol-5-on werden in 158 Teilen Wasser unter Zugabe von 28 Teilen 30-%-iger Natriumhydroxid-Lösung gelöst und durch Zugabe von Wasser auf ein Volumen von 400 Teilen gestellt. Dieser Lösung fügt man die oben hergestellte Diazosuspension hinzu und stellt mit 30-%-iger Natriumhydroxid-Lösung auf pH 5. Nach beendeter Kupplung wird die Farbstofflösung auf 70° erwärmt und durch Zugabe von 13 Teilen 30-%-iger Natriumhydroxid-Lösung auf pH 6 bis 7 gestellt, wobei die Temperatur bei 70° gehalten wird. Man lässt 20 Minuten bei pH 6 bis 7 rühren und setzt dann 35 Teile Natriumchlorid hinzu. Anschliessend wird die Suspension filtriert und im Vakuum bei 70 bis 80° getrocknet. Man erhält ein gelbes Pulver, welches sich in Wasser löst und Wolle aus essigsaurem Bad in sehr nassechten, gelben Tönen färbt. Die Ausbeute über alle Stufen beträgt 85 %.

**Beispiel 4:**

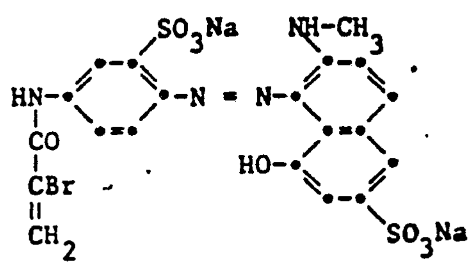

53 Teile 1,4-Phenylendiamin-2-sulfonsäure werden in 80 Teilen Wasser verrührt, auf 50° erwärmt und durch Zugabe vom wässriger Natriumhydroxid-Lösung bei pH 8 gelöst. Das Volumen wird auf 400 Teile gestellt. Nach Zugabe von ca. 180 Teilen Eis werden der Lösung bei einer Temperatur von 0 bis 3° 47 Teile Natriumchlorid und anschliessend 15 Teile Natriumacetat sowie 20 Teile Natriumhydrogencarbonat unter Rühren zugesetzt. Danach werden 67 Teile 2,3-Dibrompropionsäurechlorid portionenweise zugegeben. Durch Zugabe von Eis wird eine Temperatur von 5 bis 10° eingehalten. Anschliessend wird mit Wasser auf ein Volumen von 800 Teilen eingestellt und bei ca. 30° unter starkem Rühren 60 Teile 32-%-ige Salzsäure zugesetzt. Bei adiabatischer Temperaturführung wird mit 62,5 Teilen 4N Natriumnitrit-Lösung diazotiert. Ein eventuell vorhandener Nitritüberschuss kanm mit Sulfaminsäure zerstört werden.

70 Teile 2-N-Methylamino-8-hydroxynaphthalin-6-sulfonsäure werden in 100 Teilen Wasser unter Zugabe von 27,5 Teilen 30-%-iger Natriumhydroxid-Lösung gelöst und durch Zugabe von Wasser auf ein Volumen von 350 Teilen gestellt. Diese Lösung fügt man der oben hergestellten Diazosuspension hinzu und stellt mit 30-%-iger Natriumhydroxid-Lösung auf pH 3,8. Zur Vervollständigung der Kupplung werden dann 15 Teile Natriumacetat zugegeben. Nach beendeter Kupplung wird die Farbstofflösung durch Zugabe von 50 Teilen 30-%-iger Natriumhydroxid-Lösung auf pH 11,5 bis 12 gestellt, wobei die Temperatur zwischen 25 und 30° gehalten wird. Man lässt 15 Minuten bei pH 11,5 bis 12 rühren und neutralisiert dann auf pH 6 durch Zugabe von 32-%-iger Salzsäure. Anschliessend wird die Suspension filtriert und im Vakuum bei 70 bis 80° getrocknet. Man erhält ein blaurotes Pulver, welches sich in Wasser löst und Wolle aus essigsaurem Bad in sehr nassechten, blaustichig roten Tönen färbt. Die Ausbeute über alle Stufen beträgt 90 %.

Wenn man wie in den Beispielen 1 bis 4 angegeben verfährt, als Phenylendiamin 1,3-Phenylendiamin-4-sulfonsäure, als Acylierungsmittel die in Spalte 1 der Tabelle angegebenen Acylierungsmittel in äquimolarer Menge und als Kupplungskomponente die in Spalte 2 der Tabelle angegebenen Kupplungskomponenten in äquimolarer Menge verwendet, so erhält man ebenfalls reinere Farbstoffe, als nach den bekannten Methoden. In Spalte 3 der Tabelle ist die Nuance der erhaltenen Färbungen auf Wolle angegeben.

**Tabelle**

| Beispiel | Acylierungsmittel | Kupplungskomponente | Nuance |
|---|---|---|---|
| 5 | 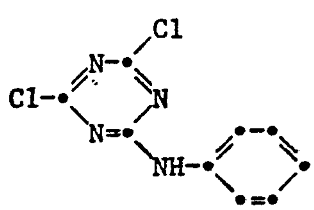 | 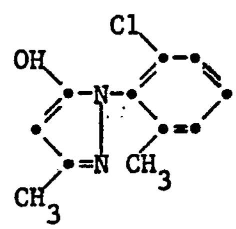 | gelb |
| 6 | $Cl-CO-CHCl-CH_2Cl$ | 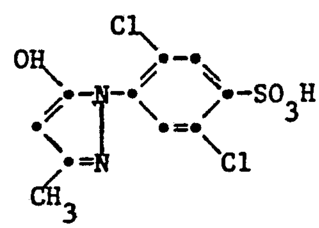 | gelb |
| 7 | $Cl-CO-CHCl-CH_2Cl$ | 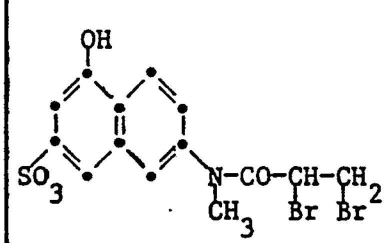 | orange |

**Tabelle** (Fortsetzung)

| Beispiel | Acylierungsmittel | Kupplungskomponente | Nuance |
|---|---|---|---|
| 8 | $Cl-CO-CCl=CH_2$ | 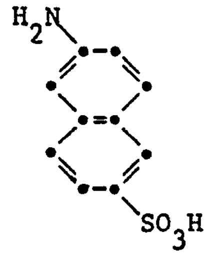 | orange |
| 9 | $Cl-CO-CHBr-CH_2Br$ | 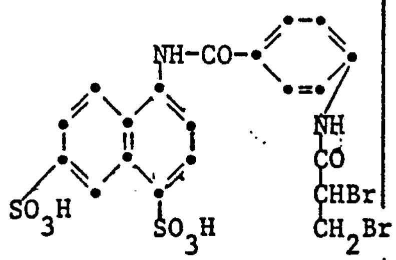 | rot |
| 10 | $Cl-CO-CHBr-CH_2Br$ | 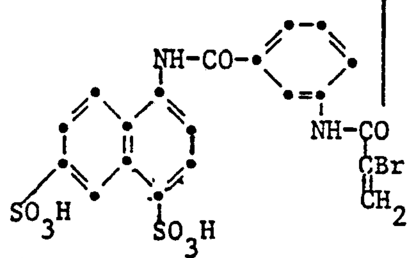 | rot |
| 11 | $Cl-CO-CHBr-CH_2Br$ | 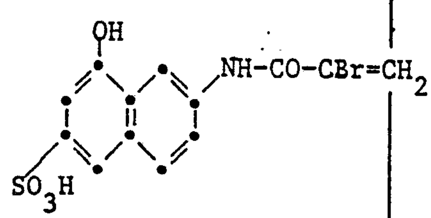 | rot |
| 12 | $Cl-CO-CHBr-CH_2Br$ | 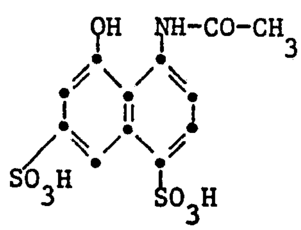 | rot |

**Tabelle** (Fortsetzung)

| Beispiel | Acylierungsmittel | Kupplungskomponente | Nuance |
|---|---|---|---|
| 13 | 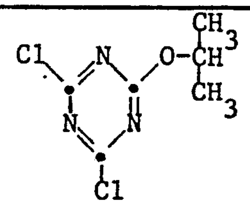 | 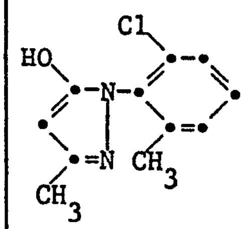 | gelb |
| 14 | 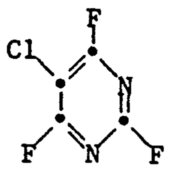 | 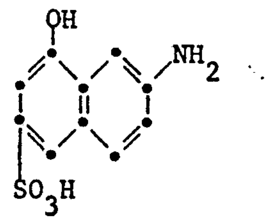 | blaurot |
| 15 | 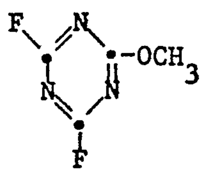 | 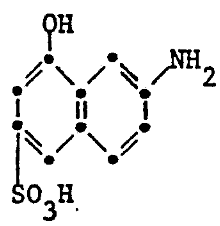 | blaurot |

**Beispiel 16:**

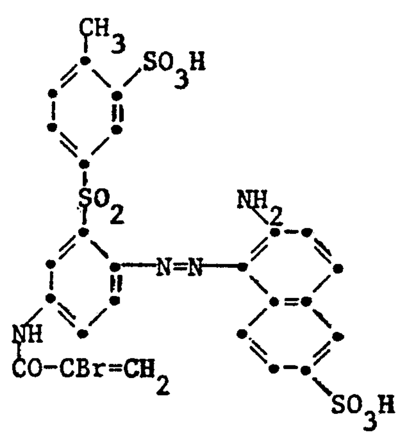

37,6 Teile 2,5-Diamino-4'-methyl-1,1'-diphenylsulfon-3'-sulfonsäure weden in 400 Teilen Wasser bei 40° mit 11,5 Teilen einer 30-%-igen Natriumhydroxydlösung auf pH 7,5 klar gelöst. Man versetzt mit 25 Teilen Natriumchlorid und 13 Teilen Natriumhydrogencarbonat, kühlt auf 0 bis 5° und lässt langsam unter intensivem Rühren 30 Teile α,β-Dibrompropionylchlorid einlaufen. Der pH am Ende der Acylierung beträgt ca. 6,5. Die Acylierungssuspension wird mit Eis und Wasser auf 600 Teile gestellt und mit 120 Teilen α-Naphthalinsulfonsäure 2,5 N versetzt. Bei 5 bis 10° wird mit 25 Teilen 4N Natriumnitritlösung diazotiert. Ein eventuell vorhandener Nitritüberschuss wird mit Sulfaminsäure zerstört. 40 Teile Natriumacetat werden zugesetzt.

Eine Suspension bestehend aus 23,5 Teilen 2-Amino-naphthalin-6-sulfonsäure in 1000 Teilen Wasser von 40° und mit 10,5 Teilen 10N Natronlauge auf pH 7 bis 7,5 gestellt und dann mit Eis auf ein Volumen von 150 Teilen gestellt, wird in die Diazosuspension eingerührt. Mit ca. 35 Teilen 10N Natronlauge wird der pH der Kupplungsmasse auf 7 gestellt. Dann stellt man mit 12 Teilen 10N Natronlauge auf pH 11,5 bis 12. Nach 15 Minuten wird mit 2 Teilen 10N Salzsäure auf pH 8 gestellt, der ausgefallene Farbstoff abgetrennt, mit 3-%-iger Sole gewaschen und im Vakuum bei 80° getrocknet. Es werden 70 Teile eines braunroten Pulvers erhalten, welches in Wasser gelöst aus essigsaurem Bad Wolle in sehr nassechtem gelbstichig-roten Farbton färbt.

## Patentansprüche

1. Verfahren zur Herstellung von Azoverbindungen der Formel

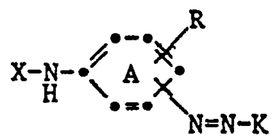

(1),

worin K der Rest einer Kupplungskomponente, R eine wasserlöslichmachende Gruppe und X ein faserreaktiver Rest ist, und der Benzolring A gegebenenfalls weitersubstituiert ist, dadurch gekennzeichnet, dass man eine Verbindung der Formel

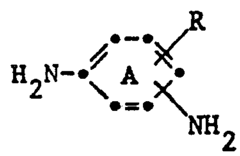

(2)

in wässrigem Medium, enthaltend 5 bis 25 Gewichtsprozent Alkalihalogenid oder Alkalisulfat, bezogen auf das Gewicht des Mediums, mit einer den Rest X einführenden Verbindung zu der Verbindung der Formel

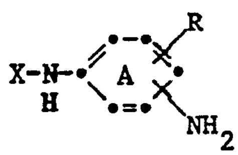

(3)

umsetzt, diese diazotiert und auf eine Kupplungskomponente der Formel

H - K (4)

kuppelt, wobei A, R, X und K in den Formeln (2), (3) und (4) die unter Formel (1) angegebenen Bedeutungen haben.

2. Verfahren gemäss Anspruch 1, dadurch gekennzeichnet, dass das Gewichtsverhältnis von Wasser zu der Verbindung der Formel (2) 3 : 1 bis 20 : 1, insbesondere 4 : 1 bis 8 : 1 ist.

3. Verfahren gemäss Anspruch 1, dadurch gekennzeichnet, dass man 8 bis 13 Gewichtsprozent Alkalihalogenid oder Alkalisulfat verwendet.

4. Verfahren gemäss Anspruch 3, dadurch gekennzeichnet, dass man Natriumchlorid verwendet.

5. Verfahren gemäss Anspruch 1, dadurch gekennzeichnet, dass man in Gegenwart weiterer zusätzlicher Salze acyliert, insbesondere ein gepuffertes Gemisch von Salzen verwendet.

6. Verfahren gemäss Anspruch 5, dadurch gekennzeichnet, dass man eine Mischung von Alkalicarbonat oder Alkalihydrogencarbonat, Alkaliacetat und Alkalihalogenid oder Alkalisulfat verwendet.

7. Verfahren gemäss Anspruch 1, dadurch gekennzeichnet, dass man eine Verbindung der Formel (2) mit einer den Rest X einführenden Verbindung bei 0° bis 20° C und einem pH-Wert von 3 bis 9 umsetzt.

8. Verfahren gemäss Anspruch 1, dadurch gekennzeichnet, dass man eine Verbindung der Formel (2) verwendet, wobei R eine Sulfophenylsulfon- oder insbesondere eine Sulfonsäuregruppe ist.

9. Verfahren gemäss Anspruch 8, dadurch gekennzeichnet, dass man als Verbindung der Formel (2) 2,5-Diamino-4'-methyl-1,1'-diphenylsulfon-3'-sulfonsäure oder 1,3-Phenylendiaminsulfonsäure, 1,4-Phenylendiamin-2-sulfonsäure verwendet.

10. Verfahren gemäss Anspruch 1, dadurch gekennzeichnet, dass man als den Rest X einführende Verbindung α,β-Dibrompropionsäurechlorid, α,β-Dichlorpropionsäurechlorid, α-Chloracrylsäurechlorid, Chloracetylchlorid oder Chloressigsäureanhydrid verwendet.

11. Verfahren gemäss Anspruch 1 dadurch gekennzeichnet dass man als Kupplungskomponente der Formel (4) eine Kupplungskomponente der Benzol-, Naphthalin- oder der heterocyclischen Reihe verwendet.

12. Verfahren gemäss Anspruch 1 zur Herstellung der Azoverbindung der Formel

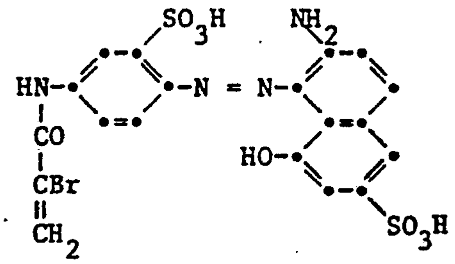

(5),

dadurch gekennzeichnet, dass man 1,4-Phenylendiamin-2-sulfonsäure mit α,β-Dibrompropionsäurechlorid umsetzt und die erhaltene Verbindung ohne Isolierung direkt diazotiert und auf 2-Amino-8-hydroxynaphthalin-6-sulfonsäure kuppelt und anschliessend mit einem bromwasserstoffabspaltenden Mittel umsetzt.

13. Verfahren gemäss Anspruch 1 zur Herstellung der Azoverbindung der Formel

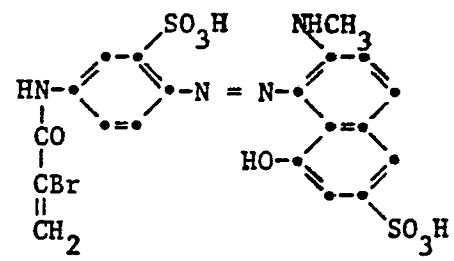

(6),

dadurch gekennzeichnet, dass man 1,4-Phenylendiamin-2-sulfonsäure mit α,β-Dibrompropionsäurechlorid umsetzt und die erhaltene Verbindung ohne Isolierung direkt diazotiert und auf 2-N-Methylamino-8-hydroxy-naphthalin-6-sulfonsäure kuppelt und anschliessend mit einem bromwasserstoff-abspaltenden Mittel umsetzt.

14. Verfahren gemäss Anspruch 1 zur Herstellung des Azofarbstoffes der Formel

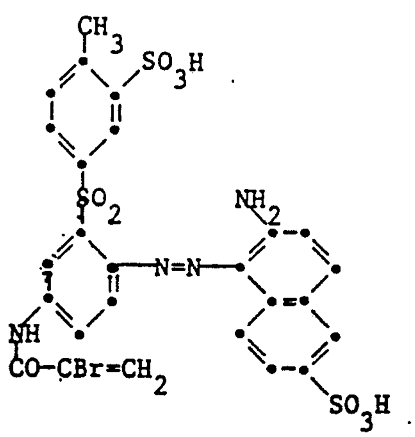

dadurch gekennzeichnet, dass man 2,5-Diamimo-4'-methyl-1,1'-diphenylsulfon-3'-sulfonsäure mit α,β-Dibrompropionsäurechlorid umsetzt und die erhaltene Verbindung ohne Isolierung oder Reinigung direkt diazotiert und auf 2-Aminonapthalin-6-sulfonsäure kuppelt und anschliessend mit einem bromwasserstoffabspaltenden Mittel umsetzt.

15. Verfahren zur Herstellung von Verbindungen der Formel

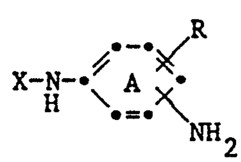

(3),

worin X ein faserreaktiver Rest und R eine wasserlöslichmachende Gruppe ist und der Benzolring A gegebenenfalls weitersubstituiert ist, dadurch gekennzeichnet, dass man eine Verbindung der Formel

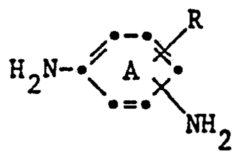

(2)

in wässrigem Medium, enthaltend 5 bis 25 Gewichtsprozent Alkalihalogenid oder Alkalisulfat, bezogen auf das Gewicht des Mediums, mit einer den Rest X einführenden Verbindung zu der Verbindung der Formel (3) umsetzt.

## Claims

1. A process for the preparation of an azo compound of the formula

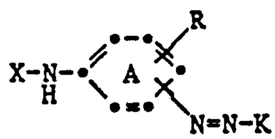

(1)

in which K is the radical of a coupling component, R is a water-solubilising group and X is a fibre-reactive radical, and the benzene ring A may be further substituted, which process comprises reacting a compound of the formula

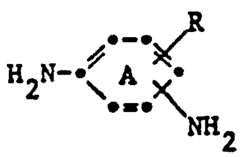

(2)

in an aqueous medium containing 5 to 25 per cent by weight of an alkali metal halide or alkali metal sulfate, based on the weight of the medium, with a compound which introduces the radical X to give the compound of the formula

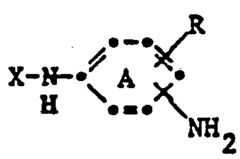

(3)

diazotising this compound and coupling the product with a coupling component of the formula

H - K (4)

in which formulae (2), (3) and (4) A, R, X and K are as defined for formula (1).

2. A process according to claim 1, wherein the weight ratio of water to the compound of the formula (2) is from 3 : 1 to 20 : 1, in particular from 4 : 1 to 8 : 1.

3. A process according to claim 1, which comprises the use of 8 to 13 per cent by weight of an alkali metal halide or alkali metal sulfate.

4. A process according to claim 3, which comprises the use of sodium chloride.

5. A process according to claim 1, which comprises carrying out the acylation in the presence of further additional salts, in particular using a buffered mixture of salts.

6. A process according to claim 5, which comprises the use of a mixture of an alkali metal carbonate or alkali metal bicarbonate, an alkali metal acetate and an alkali metal halide or alkali metal sulfate.

7. A process according to claim 1, where a compound of the formula (2) is reacted with a compound which introduces the radical X at 0° to 20° C and at a pH of from 3 to 9.

8. A process according to claim 1, which comprises the use of a compound of the formula (2) in which R is a sulfophenylsulfone or, in particular, a sulfonic acid group.

9. A process according to claim 8, wherein the compound of the formula (2) is 2,5-diamino-4'-methyl-1,1'-diphenylsulfone-3'-sulfonic acid or 1,3-phenylenediaminesulfonic acid or 1,4-phenylenediamine-2-sulfonic acid.

10. A process according to claim 1, wherein the compound which introduces the radical X is a α,β-dibromopropionyl chloride, α,β-dichloropropionyl chloride, α-chloroacryloyl chloride, chloroacetyl chloride or chloroacetic anhydride.

11. A process according to claim 1, wherein the coupling component of the formula (4) is a coupling component of the benzene, naphthalene or heterocyclic series.

12. A process according to claim 1 for the preparation of the azo compound of the formula

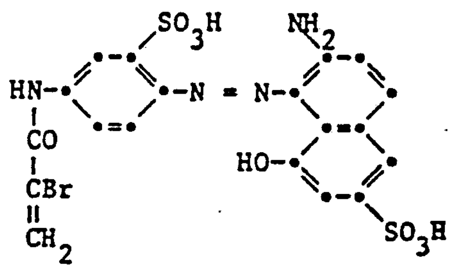

(5)

which comprises reacting 1,4-phenylenediamine-2-sulfonic acid with α,β-dibromopropionyl chloride, diazotising the resultant compound directly without isolation, coupling the product with 2-amino-8-hydroxynaphthalene-6-sulfonic acid and subsequently reacting the coupling product with a dehydrobrominating agent.

13. A process according to claim 1 for the preparation of the azo compound of the formula

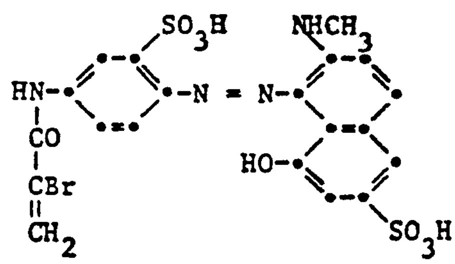

(6)

which comprises reacting 1,4-phenylenediamine-2-sulfonic acid with α,β-dibromopropionyl chloride, diazotising the resultant compound directly without isolation, coupling the product with 2-N-methylamino-8-hydroxy-naphthalene-6-sulfonic acid and subsequently reacting the coupling product with a dehydrobrominating agent.

14. A process according to claim 1 for the preparation of the azo dye of the formula

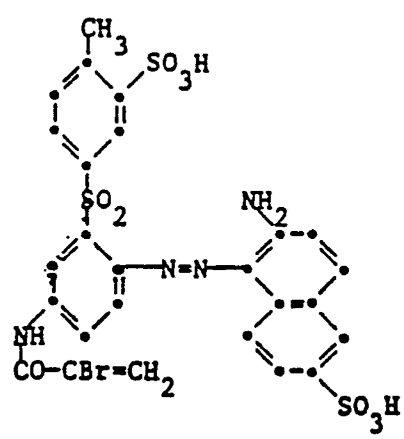

which comprises reacting 2,5-diamino-4'-methyl-1,1'-diphenylsulfone-3'-sulfonic acid with α,β-dibromopropionyl, chloride, diazotising the resultant compound directly without isolation or purification, coupling the product with 2-aminonaphthalene-6-sulfonic acid and subsequently reacting the coupling product with a dehydrobrominating agent.

15. A process for the preparation of a compound of the formula

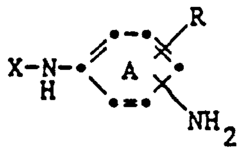

(3)

in which X is a fibre-reactive radical and R is a water-solubilising group, and the benzene ring A may be further substituted, which process comprises reacting a compound of the formula

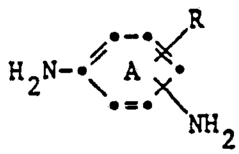

(2)

in an aqueous medium containing 5 to 25 per cent by weight of an alkali metal halide or alkali metal sulfate, based on the weight of the medium, with a compound which introduces the radical X to give the compound of the formula (3).

**Revendications**

1. Procédé pour la préparation de composés azoïques de formule

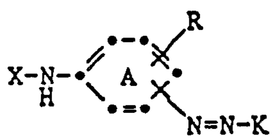

(1),

dans laquelle K est le reste d'un composant de copulation, R est un groupe conférant la solubilité dans l'eau, et X est un reste réactif avec les fibres, et le noyau benzénique A est éventuellement substitué davantage, caractérisé en ce que l'on fait réagir un composé de formule

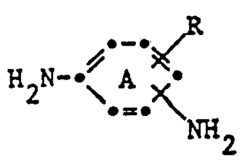

(2)

en milieu aqueux contenant de 5 à 25 % en poids d'un halogénure alcalin ou d'un sulfate alcalin, par rapport au poids du milieu, avec un composé introduisant le reste X, pour aboutir au composé de formule

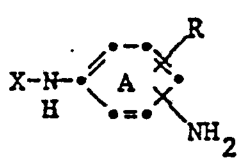

(3)

on soumet celui-ci à une diazotation et on le fait copuler sur un composant de copulation de formule

H - K (4),

A, R, X et K, dans les formules (2), (3) et (4), ayant les significations données sous la formule (1).

2. Procédé selon la revendication 1, caractérisé par le fait que le rapport pondéral de l'eau au composé de formule (2) est de 3 : 1 à 20 : 1, en particulier de 4 : 1 à 8 : 1.

3. Procédé selon la revendication 1, caractérisé par le fait que l'on utilise de 8 à 13 % en poids d'halogénure alcalin ou de sulfate alcalin.

4. Procédé selon la revendication 3, caractérisé par le fait que l'on utilise le chlorure de sodium.

5. Procédé selon la revéndication 1, caractérisé par le fait que l'on effectue l'acylation en présence d'autres sels supplémentaires, en particulier on utilise en mélange de sels tamponné.

6. Procédé selon la revendication 5, caractérisé par le fait que l'on utilise un mélange de carbonate alcalin ou d'hydrogénocarbonate alcalin, d'acétate alcalin et d'halogénure alcalin ou sulfate alcalin.

7. Procédé selon la revendication 1, caractérisé par le fait que l'on fait réagir un composé de formule (2) avec un composé introduisant le reste X, à 0 - 20° C et à un pH de 3 à 9.

8. Procédé selon la revendication 1, caractérisé par le fait que l'on utilise un composé de formule (2) dans lequel R est un groupe sulfophénylsulfonyle ou, en particulier, un groupe sulfo.

9. Procédé selon la revendication 8, caractérisé par le fait qu'en tant que composé de formule (2), on utilise l'acide 2,5-diamino-4'-méthyl-1,1'-diphénylsulfonyl-3'-sulfonique ou l'acide 1,3-phénylène-diaminesulfonique, l'acide 1,4-phénylènediamine-2-sulfonique.

10. Procédé selon la revendication 1, caractérisé par le fait qu'en tant que composé introduisant le reste R, on utilise le chlorure d'α,β-dibromopropionyle, le chlorure d'α,β-dichloropropionyle, le chlorure d'α-chloro-acroyle, le chlorure de chloracétyle ou l'anhydride chloracétique.

11. Procédé selon la revendication 1, caractérisé par le fait qu'en tant que composant de copulation de formule (4), on utilise un composant de copulation de la série du benzène, du naphtalène ou de la série hétérocyclique.

12. Procédé selon la revendication 1, pour la préparation du composé azoïque de formule

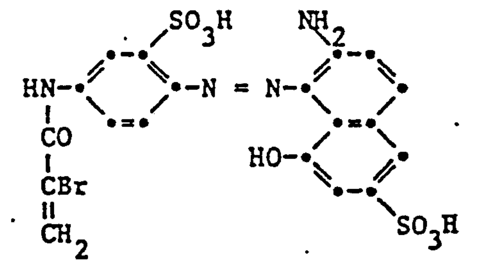

(5) ,

caractérisé par le fait que l'on fait réagir l'acide 1,4-phénylènediamine-2-sulfonique avec le chlorure d'α,β-dibromopropionyle, et on soumet directement à une diazotation le composé obtenu, sans l'isoler, et on le fait copuler sur l'acide 2-amino-8-hydroxynaphtalène-6-sulfonique et ensuite on le fait réagir avec un agent éliminant l'acide bromhydrique.

13. Procédé selon la revendication 1 pour la préparation du composé azoïque de formule

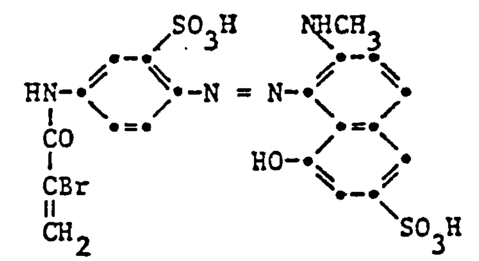

(6),

caractérisé par le fait que l'on fait réagir l'acide 1,4-phénylènediamine-2-sulfonique avec le chlorure d'α,β-dibromopropionyle, et on soumet directement à une diazotation le composé obtenu, sans l'isoler, et on le fait copuler sur l'acide 2-N-méthylamino-8-hydroxynaphtalène-6-sulfonique et ensuite on le fait réagir avec un agent éliminant l'acide bromhydrique.

14. Procédé selon la revendication 1, pour la préparation du colorant azoïque de formule

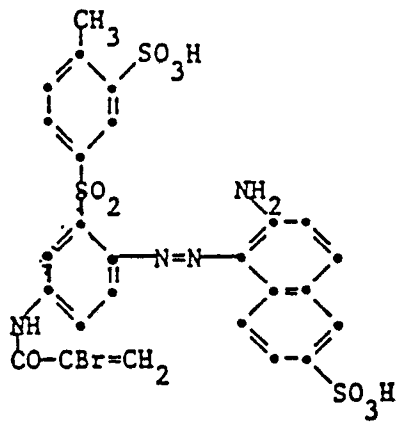

caractérisé par le fait que l'on fait réagir l'acide 2,5-diamino-4'-méthyl-1,1'-diphénylsulfonyl-3'-sulfonique avec le chlorure d'α,β-dibromopropionyle, et on soumet directement à une diazotation le composé obtenu, sans isolement ni purification, et on le fait sur l'acide 2-aminonaphtalène-6-sulfonique et ensuite on le fait réagir avec un agent éliminant l'acide bromhydrique.

15. Procédé pour la préparation de composés de formule

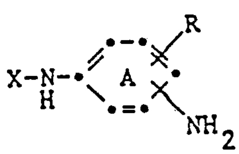

(3),

dans laquelle X est un reste réactif avec les fibres et R est un groupe conférant la solubilité dans l'eau, et le noyau benzénique A est éventuellement substitué davantage, caractérisé en ce que l'on fait réagir un composé de formule

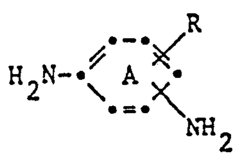

(2)

en milieu aqueux contenant de 5 à 25 % en poids d'un halogénure alcalin ou d'un sulfate alcalin, par rapport au poids du milieu, avec un composé introduisant le reste X, pour aboutir au composé de formule (3).